**Europäisches Patentamt**

**European Patent Office**

**Office européen des brevets**

(19)

(11) Veröffentlichungsnummer: **0 206 077**
**A2**

(12) # EUROPÄISCHE PATENTANMELDUNG

(21) Anmeldenummer: **86107844.2**

(22) Anmeldetag: **09.06.86**

(51) Int. Cl.⁴: **B 03 C 1/28,** G 01 N 33/553

(30) Priorität: **22.06.85 DE 3522365**

(43) Veröffentlichungstag der Anmeldung: **30.12.86**
**Patentblatt 86/52**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Müller-Ruchholtz, Wolfgang, Prof. Dr. Dr., Tränkenberg 8, D-2300 Molfsee (DE)**
Erfinder: **Kandzia, Jörg, Dipl. Biol., Bruckmühlenweg 11, D-7815 Kirchzarten-Bruckmühle (DE)**
Erfinder: **Haas, Wolfgang, Dipl.-Bio.-Chem., Hallgrafenstrasse 17, D-8230 Bad Reichenhall (DE)**
Erfinder: **Leyhausen, Gabriele, Dr., Westring 279, D-2300 Kiel 1 (DE)**

(54) **Trenngerät für magnetische Partikel aus flüssiger Phase.**

(57) Die Erfindung betrifft ein Trenngerät zum Abtrennen von magnetischen Partikeln aus flüssigen Phasen, bestehend aus einer Auftragungseinheit, einer Trenneinheit und einer Sammeleinheit. Dieses Trenngerät kann eingesetzt werden zum Auftrennen von biologischen Materialien, insbesondere zum Abtrennen von Zellen, Antigenen, Antikörpern, Enzymen usw. mit Hilfe von z.B. magnetischen Mikrospheres (MIMS).

EP 0 206 077 A2

BAYER AKTIENGESELLSCHAFT    5090 Leverkusen, Bayerwerk
Konzernverwaltung RP
Patentabteilung    Bu/Kü-c

## Trenngerät für magnetische Partikel aus flüssiger Phase

Die Erfindung betrifft ein Trenngerät zum Abtrennen von magnetischen Partikeln aus flüssigen Phasen bestehend aus einer Auftragungseinheit, einer Trenneinheit und einer Sammeleinheit. Dieses Trenngerät kann eingesetzt werden zum Auftrennen von biologischen Materialien, insbesondere zum Abtrennen von Zellen, Antigenen, Antikörpern, Enzymen usw. mit Hilfe von magnetischen Microspheres (MIMS).

In den letzten Jahren ist das Interesse an leistungsfähigen analytischen wie präparativen Zelltrennverfahren beträchtlich gestiegen. Da das Augenmerk des Zellbiologen zunehmend auf die Untersuchung des funktionellen Zusammenspiels auch kleinster Subpopulationen von Zellen innerhalb heterogener Gemische gerichtet ist, ist für eine Trenntechnik neben der notwendigen quantitativen physikalischen Leistungsfähigkeit die qualitativ spezifische Unterscheidungsfähigkeit zu fordern. Für die erstgenannte Anforderung hat bis in die Gegenwart immer noch die Dichtezentrifugationstechnik, die auf der Basis der physikali-

Le A 23 798 -Ausland

schen Parameter Volumen und Dichte verschiedene Zellen trennt, ihren unbestrittenen Stellenwert (s. Hutchins, D. and C.M. Steel, in: Peters, H. Editor: Separation of cells and subcellular elements 1979; A.J. Fluks, 1981, J. Immunol. Methods 41: 225; A.J. Ulmer and Flad H.D., 1984, Immunobiol. 166:238), allerdings auf Kosten begrenzter Spezifität (Trennschärfe).

Für die zweite Forderung können nur Techniken zum Einsatz kommen, die durch ihre Fähigkeit, unterschiedliche molekulare Oberflächenstrukturen der verschiedenen Zellen (Targetmoleküle wie Antigene, Rezeptoren o.ä.) als Trennungsparameter nutzen zu können, biospezifisch und damit selektiv trennen. Diesbezüglich sind besonders hervorzuheben "solid-phase" Affinitätstechniken, die auf der Grundlage serologischer Reaktionen trennen.

Durch die Fixierung von Antikörpern an Plastikoberflächen (z.B. Panningverfahren: Wysocki, L.J. and V.L. Sato, 1978, Proc. Natl. Acad. Sci. 75:2844,; Basch, R.S. et al., 1983, J. Immunol. Methods 56:269), Glas oder Gelmaterialien (Basch, R.S. et al., ebd.), die zu Säulen gepackt werden können, erlauben diese Methoden, Zellen oder zelluläres Material durch Unterscheidung von molekularen Oberflächenstrukturen, z.B. Antigenen, spezifisch zu trennen. Nachteile dieser Verfahren sind unspezifische Bindungsreaktionen mit Materialoberflächen, das Verstopfen von Säulen durch aggregierende Partikeln bzw. Zellen, Beeinträchtigung empfindlicher Zellen durch Scherkräfte, lange Prozeßzeiten und ganz besonders die mangelnde Kapazität, große Mengen zu bewältigen.

Demgegenüber können unspezifische Haftung, ungenügende Zellschonung und Scherkräfte in Flüssigphasen-Verfahren, in denen der eigentliche Trennungsvorgang aus verdünnten Suspensionen erfolgt, fast gänzlich ausgeschaltet werden. Hier sind zu nennen: Zellimmunelektrophorese (van Oss et al., 1979, Immunol. Communic. 8:419), FACS (Loken, M.R. and A.M. Stall, 1982, J. Immunol. Methods 50: R 85), und magnetische Zelltrenntechniken auf der Basis Antikörperkonjugierter magnetischer Microspheres (MIMS). Gemeinsam ist allen diesen Techniken die Kombination der serologischen Spezifität von Antikörpern mit einer steuerbaren physikalischen Kraft: elektrisches Feld, optische Erkennung oder Magnetfeld.

Obgleich z.B. der FACS gegenwärtig auf analytischem Gebiet - trotz seiner hohen Anschaffungs- und Unterhaltungs-Kosten insbesondere wegen seines multifaktoriellen Charakters - den Stand der Technik repräsentiert, hat er wie auch die Zellimmunelektrophorese einen gewichtigen Nachteil: Für den präparativen Einsatz steht eine eng begrenzte Kapazität zur Verfügung, die zur Bewältigung großer Zellmengen in angemessener Zeit nicht ausreicht.

Demgegenüber bietet der Einsatz magnetischer Microspheres bemerkenswerte Vorteile, da magnetische Separationsverfahren mit Microspheres billig und einfach im Prozeßverlauf sind und insbesondere auf präparativem Gebiet nicht nur zur Bewältigung großer Zellmengen ($10^6$ - $10^{12}$ Zellen), sondern auch zur schnellen Isolierung anderer zellulärer Partikeln wirtschaftlich angemessen eingesetzt werden können.

Le A 23 798

In diesem Zusammenhang muß aber auch als wichtiges Systemziel die parallele Entwicklung und Bereitstellung einer kostengünstigen apparativen Einheit stehen, in der technisch zuverlässig und reproduzierbar die spezifische Trennleistung von MIMS umgesetzt und die umfassenden analytischen wie präparativen Möglichkeiten flexibel und mengenwirksam nutzbar gemacht werden. Die Bereitstellung eines solchen Trenngerätes ist Gegenstand der vorliegenden Erfindung.

Die Erfindung betrifft ein Trenngerät für die magnetische Abtrennung von Partikeln. Dies sind insbesondere biologische Partikeln, vor allem Zellen, aber auch molekulare Strukturen wie z.B. Enzyme, Antigene, Antikörper oder andere biologische Substanzen, welche (a) vorher an eine magnetisierbare Komponente, wie z.B. magnetische Microspheres, affin oder anders biochemisch gebunden werden, (b) eine magnetisierbare Komponente sekundär aufgenommen haben oder (c) natürlicherweise eine molekulare Struktur beinhalten die magnetisch beeinflußbar ist. Die Abtrennung erfolgt dabei aus flüssiger Phase in einem Durchflußsystem. Dabei werden solche Partikel, die die magnetisierbare Komponente nach (a) gebunden, nach (b) aufgenommen oder nach (c) enthalten, im Magnetfeld festgehalten (positive Selektion), während gleichzeitig alle übrigen Partikeln mit dem Flüssigkeitsstrom durchlaufen und gesammelt werden (negative Selektion).

Le A 23 798

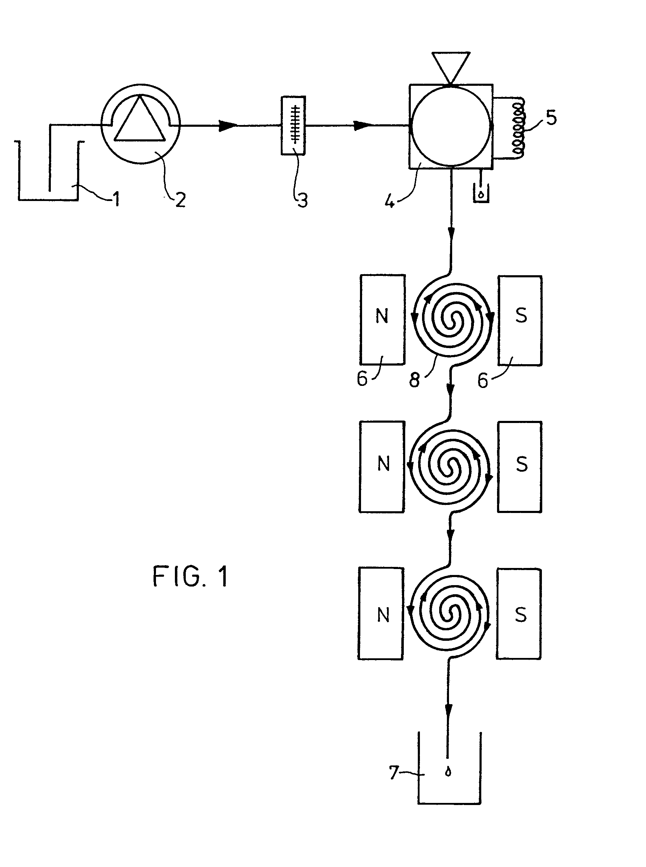

Wie die Fig. 1 zeigt, betrifft die Erfindung ein Trenngerät für die magnetische Abtrennung von magnetisierbaren Partikeln, dessen Auftragseinheit aus einem Rheodyneventilsystem(4) mit Probenschlinge (5), einer Peristaltikpumpe (2) und einem Kompaktströmungsmesser (3) besteht oder aus der Kombination mehrerer dieser Einzelelemente. Die Auftragungseinheit ist ausgelegt für die Eingabe der Probenvolumina 1 µl - 1000 ml, insbesondere 100 µl - 10 ml Probenschlingenvolumen. Das Probenaufgabeventil ist ein Mehrwegventil und besteht aus amagnetischem Material, die Probenschlinge ebenfalls aus amagnetischem Material, z.B. Edelstahl oder Teflonschlauch. Die Probenschlinge ist so positioniert, daß nach Probenaufgabe die gesamte Probe mittels vorgeschalteter Pumpeneinheit in die Trenneinheit eingebracht wird. Dabei entnimmt eine Pumpe, wie z.B. eine Peristaltikpumpe, aus einem vorgeschalteten Vorratsgefäß (1) Vorratspuffer, Spülpuffer o.ä., der mittels der Pumpe durch die Trenneinheit, je nach Aufgabeventilstellung direkt oder über die Probenschlinge gepumpt wird.

Die Peristaltikpumpe produziert stufenlos regulierbare Durchflußraten, die mittels des eingebauten Kompaktströmungsmessers meß- und kontrollierbar sind.

Die Wahl der Durchflußrate hängt ab vom Gesamtvolumen der Trenneinheit, dem Volumen der Probe und allgemein von der physikalischen und chemischen Beschaffenheit der Probe, wie zum Beispiel der Partikelzahl und der Viskosität. Als geeignet erwiesen sich Durchflußgeschwindigkeiten von 0,2 - 20 ml/min, insbesondere 0,4 ml - 4 ml/min, üblicherweise 0,4 - 1 ml/min.

Le A 23 798

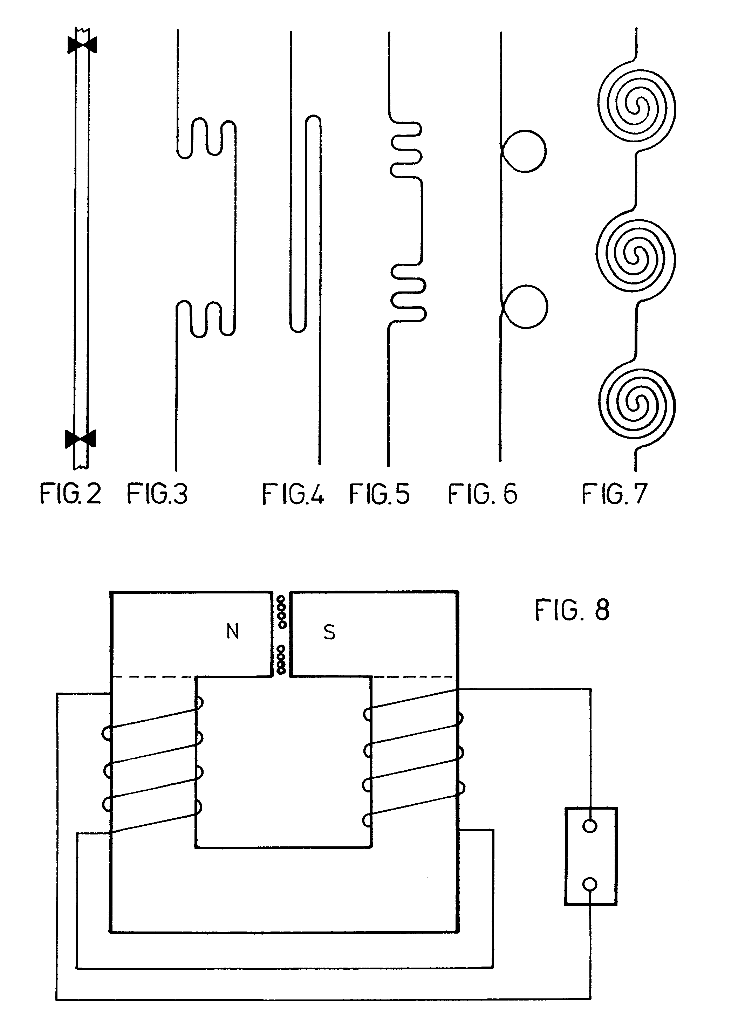

Weiterhin betrifft die Erfindung ein Trenngerät für die magnetische Abtrennung von magnetisierbaren Partikeln, dessen Trenneinheit ganz allgemein aus speziell geformten Kammern (8) verschiedenster Größe und Volumina aus Glas, mit und ohne Beschichtung (z.B. Silikon), oder aus Kunststoffen, insbesondere Teflon und seinen Derivaten, besteht. Verschiedene Ausführungsformen der Trenneinheit werden in den Fig. 2-7 gezeigt.

Diese Trenneinheit besteht aus einem oder mehreren Elementen (1-20, vorteilhafterweise 1-6, insbesondere 3 Elementen), die insgesamt aus einem oder mehreren einzelnen Stücken bevorzugt aus Teflonschlauch der Länge 1-200 cm, insbesondere 1-30 cm, mit unterschiedlichem Durchmesser (0,1-6 mm, insbesondere 0,1-2 mm) gefertigt sind und in variabler Anzahl in einem Stecksystem zusammengefügt sein können.

Bevorzugt ist die in Fig. 7 dargestellte "Trennschnecke" aus Teflonschlauch. Sie ist mechanisch und chemisch inert, formbar und dabei eigenstabil, autoklavierbar und sterilisierbar. Sie ist so konstruiert, daß sie eine optimale Positionierung im Magnetfeld erlaubt mit einer maximal dem Magnetfeld zugewandten Oberfläche bei gleichzeitiger Minimalisierung des Gesamtvolumens (Verhinderung von starken Verdünnungseffekten). Sie ist rundgewickelt und damit so gestaltet, daß sie keine Kanten und Knicke aufweist, die zur Retention von Partikeln und damit zu Systemverlusten führen würden. Sie ist also so konstruiert, daß Kalibersprünge vermieden werden, um einen gleichmäßigen, unverwirbelten "Fluß" zu gewährleisten. Sie ist so gestaltet,

Le A 23 798

daß keine Adhäsion von Partikeln stattfinden kann. Sie ist im Sinne eines allgemeinen Konstruktionsprinzips erweiterungs- und ausbaufähig.

Eine Variationsmöglichkeit für die Trenneinheit ist ihr Aufbau aus einer oder mehreren miteinander verbundenen Trennkammern. Diese Trennkammern bestehen aus Glas oder Kunststoff, insbesondere Teflon, und werden durch einen amagnetischen Metallrahmen zusammengehalten und abgedichtet. Sie sind mechanisch und chemisch inert, eigenstabil, sterilisierbar und autoklavierbar. Sie sind so konstruiert, daß sie eine optimale Positionierung im Magnetfeld erlauben, mit einer maximal dem Magnetfeld zugewandten Oberfläche bei gleichzeitiger Minimalisierung des Gesamtvolumens.

Ein wesentlicher Teil der Trenneinheit sind die Elektromagneten (6), die in einer bevorzugten Ausführungsform stufenlos regelbar sind.

Bei der Verwendung von mehreren Magneten ist es vorteilhaft, jedes für sich über ein separates Netzgerät mit Strom zu versorgen und zu steuern.

Die erreichbaren Feldstärken umfassen einen Bereich von 0-40 Kilo-Gauss. Die aktuelle Feldstärke pro Magnet wird mittels einer im Feld installierten Hall-Sonde direkt abgelesen, kontrolliert und gegebenenfalls registriert.

Die Netzgeräte werden mit Wechselstrom betrieben, wobei das Netzgerät einen stabilisierten Gleichstrom oder

Le A 23 798

Wechselstrom liefert, der als Dauer- oder Impulsstrom charakterisiert ist. Der Impulsstrom hat bei variabler Frequenz, Amplitude und Pausendauer Sinus-, aber vorzugsweise Rechteck- oder Sägezahnform, die oszillographisch kontrollierbar und registrierbar ist. Die Leistung der verwendeten Netzgeräte hängt von der gewünschten Feldstärke ab, wobei letztere vom experimentellen Ansatz bestimmt wird.

Die Zahl der Elektromagneten richtet sich im wesentlichen nach Art und Form der Trenneinheit und beträgt 1-20 wobei 2 bis 6 bevorzugt werden.

Die verwendeten Elektromagnete setzen sich aus stromdurchflossenen Spulen jeglicher Form mit einem Kern aus Weicheisen, Ferrit oder anderen ferro-/paramagnetischen Stoffen zusammen (Fig. 8 zeigt eine Darstellung im Detail). Das auf die Trenneinheit einwirkende aktuelle Magnetfeld ist von der Größe des Luftspaltes zwischen Nord- und Südpol, d.h. von Länge und Dichte der dazwischen verlaufenden Feldlinien abhängig. Auf die in diesem Luftspalt befindliche Trenneinheit wirken in Abhängigkeit vom verwendeten Netzgerät, intermittierend oder konstant, homogene Magnetfelder ein.

Teil des Trenngerätes ist eine Sammmeleinheit die aus einem Fraktionssammler (7) besteht, wobei das Fraktionsvolumen über eine optische Erkennung (Photometer, Tropfenzähler) einstellbar und steuerbar ist.

Le A 23 798

0206077

Sämtliche regelbaren Funktionen können mit Hilfe von Microprozessoren automatisch geregelt werden. Das gilt für einzelne Funktionen wie auch mehrere im Verbund. Dadurch ergeben sich zum Beispiel die Möglichkeiten fortlaufender automatischer Probenaufgaben, Pufferwechsel,sowie optisch/elektronischer Kontrollen mit ein- bis mehrmaliger Ventilgesteuerter Rückführung.

Le A 23 798

## Anwendungsbeispiel

1:1 Gemische peripherer Blutlymphozyten HLA-unterschied-licher Spender (HLA-Bw6$^+$ und HLA-Bw4$^+$) werden mit MIMS inkubiert. An die MIMS wird ein monoclonaler anti-HLA Bw6 Antikörper affin gebunden, d.h. sie dienen als magnetisches Trennreagenz. Die Herstellung der MIMS und die Gewinnung der Lymphozyten erfolgt wie in der deutschen Patentanmeldung P 34 44 939.6 beschrieben.

Über ein Rheodyneventil wird eine 2 ml umfassende Probe (1-10 x 10$^6$ Zellen + 0,5-2,5 mg MIMS) in die Probenschlinge eingebracht, die über das obere Ende der Trennschnecke mit dieser verbunden ist. Danach werden drei hintereinanderliegende Elektromagnete eingeschaltet. Der obere Teil der Trennschnecke ist einem magnetischen Feld von 1,7 Kilo-Gauss (KG) ausgesetzt.

Nun wird die Zellsuspension mittels einer Peristaltikpumpe in einem kontinuierlichen "Free-flow" System mit einer Durchflußrate von 0,4 ml/min durch die Trennschnecke eluiert und somit den magnetischen Feldern ausgesetzt.

Es wird das doppelte Flüssigkeitsvolumen der Trennschnecke als Eluatfraktion gesammelt. Anschließend werden die Elektromagnete ausgeschaltet und die Magnetfraktion durch pulsartiges Ausspülen mittels der Pumpe gewonnen.

Die Trennleistung dieses Gesamtsystems setzt sich zusammen aus der biologischen Spezifität des hier verwendeten Antikörpermoleküls, der physikalischen Auswirkung des entsprechend den Versuchsbedingungen zu wählenden Magnetfeldes sowie Material, Form und Volumen der Trennsäule.

Le A 23 798

0206077

Mit dem hier beschriebenen Trenngerät sind bezüglich des Anwendungsbeispieles folgende Daten bei nur einmaligem Durchlauf erzielt worden:

Die Reinheit der Eluatfraktion beträgt zwischen 96 und 100 %, was bedeutet, daß die Magnetfelder stark genug sind, um die magnetischen Partikeln samt der daran bindenden Zellen zurückzuhalten.

Die Reinheit der Magnetfraktion beträgt 85-95 %, d.h. 5-15 % der Zellen werden unspezifisch zurückgehalten. Die Rückgewinnung der Zellen beträgt zwischen 95-100 %. Daß die Magnetfelder für diesen Versuch optimal eingestellt wurden, zeigt die kaum beeinträchtigte Vitalität der Zellen nach der Trennung, die auch dann noch zwischen 92 und 99 % liegt.

Zusammenfassend ist festzustellen, daß das Trenngerät analytisch und präparativ einsetzbar ist.

Patentansprüche

1. Trenngerät für die magnetische Abtrennung von magnetisierbaren Partikeln bestehend aus einer Auftragungseinheit (1, 2, 3, 4, 5) für das Auftragen und den Transport von Probe und Puffer, einer Trenneinheit aus Kunststoff oder Glas, (8) zum Abtrennen der magnetisierbaren Partikel versehen mit einem oder mehreren Elektromagneten (6), die durch ihre magnetische Kraft die magnetisierbaren Partikel in der Trenneinheit zurückhalten können, und einer Sammeleinheit (7).

2. Trenngerät nach Anspruch 1, dessen Auftragungseinheit aus einer Probenschlinge (5), einem Mehrwegventil (4), einer Pumpe (2) und einem Durchflußmesser (3) besteht.

3. Trenngerät nach den Ansprüchen 1 und 2, dessen Probenschlinge (5) ein Volumen von 1 µl bis 1000 ml besitzt.

4. Trenngerät nach Anspruch 1, dessen Trenneinheit (8) aus Glas oder Kunststoff mit oder ohne Beschichtung besteht.

5. Trenngerät nach den Ansprüchen 1 und 4, mit einer Trenneinheit (8) in Form von Kammern, Schläuchen oder Röhren.

Le A 23 798

6.  Trenngerät nach den Ansprüchen 1, 4 und 5, dessen Trenneinheit aus 1-20 Elementen, vorzugsweise 3 Elementen besteht.

7.  Trenngerät nach den Ansprüchen 1 und 4 bis 6, dessen Trenneinheit 8 mit einem oder mehreren Elektromagneten (6) versehen ist, von denen jeder über ein eigenes Netzgerät mit Strom versorgt wird.

8.  Trenngerät nach den Ansprüchen 1 und 4 bis 7, dessen Elektromagneten über das jeweilige Netzgerät stufenlos regelbar sind.

9.  Trenngerät nach Anspruch 1, dessen Sammeleinheit (7) ein Fraktionssammler ist.

10.  Verwendung des Trenngerätes nach den Ansprüchen 1 bis 9 zur Abtrennung von magnetischen Partikeln.

11.  Verfahren zur Abtrennung von magnetischen Partikeln, dadurch gekennzeichnet, daß das Trenngerät nach den Ansprüchen 1 bis 9 verwendet wird.

Le A 23 798

FIG. 1

0206077
2/2

FIG.2  FIG.3      FIG.4   FIG.5    FIG. 6     FIG.7

FIG. 8